# EUROPEAN PATENT APPLICATION

(11) **EP 4 154 812 A1**
(43) Date of publication of application: **29.03.2023**
(21) Application number: 21898439.1
(22) Date of filing: 10.11.2021
(51) Int. Cl.: A61B 5/15, A61B 5/153, A61B 5/00, A61B 8/08, A61B 8/00, A61M 5/42

(54) **AUTOMATIC INVASIVE DEVICE FOR BODY**

(30) Priority: 27.11.2020 KR 20200161868; 27.11.2020 KR 20200161869; 27.11.2020 KR 20200161871; 27.11.2020 KR 20200161873
(71) Applicant: Airs Medical Co., Ltd., Seoul 06142 (KR)
(72) Inventor: KANG, Seung Man, Seoul 06648 (KR); KIM, Yun Myeong, Seoul 06142 (KR); LEE, Hye Seong, Seoul 06142 (KR)
(74) Representative: Frenkel, Matthias Alexander
(86) International application number: PCT/KR2021/016335
(87) International publication number: WO 2022/114613

(57) **Abstract**

The present invention relates to an automatic invasive device for body and, more particularly, to an automatic invasive device for body comprising a body press unit capable of pressing a human body part to detect a puncturing position. The present invention also relates to an automatic invasive device for body comprising at least one of a rotatable probe unit, a vacuum tube driving unit, and a body contact material supply unit. The automatic invasive device for body according to the present invention comprises: a syringe needle unit support part that supports a syringe needle that enters the body; and a press unit (500) that presses the body that has a site to be subjected to an invasive procedure.

## Description

### TECHNICAL FIELD

The present invention relates to an automatic invasive device for body and, more particularly, to an automatic invasive device for body comprising a body press unit capable of pressing a human body part to detect a puncturing position.

The present invention also relates to an automatic invasive device for body comprising at least one of a rotatable probe unit, a vacuum tube driving unit, and a body contact material supply unit.

### BACKGROUND ART

In medical institutions such as clinics and hospitals, there are many cases in which a syringe needle is inserted into a person's superficial vein for the collection of blood or the injection of injectable solutions. Currently, doctors, nurses, or medical technologist, who have learned and been trained to locate blood vessels such as superficial veins, look for the superficial vein and insert a syringe therein. However, training takes a long time, and expenses for hiring professional human resources continue to incur. In addition, there are many cases where they do not succeed in one shot and have to insert the syringe several times. When the conditions of blood vessels are not good, such as babies, the elderly, or patients undergoing chemotherapy, or when the blood vessels are difficult to see visually because the skin is dark, it is sometimes very difficult for even a skilled specialist to find blood vessels.

In addition, even for well-educated and skilled medical staffs, repetitive blood sampling is a procedure that is highly tiring and causes a lot of rejection. Furthermore, blood collection by humans has many limitations, such as the risk that the subject's blood accidentally splashes on an operator or enters the operator's body to cause infection during the blood collection process.

In order to solve the above limitations according to the related art, the present applicant filed Korean Application Publication No. 10-2020-0010302 on January 29, 2020 for a method of automatically sensing the position of a body part to be subjected to penetration (invasive procedure), for example, a superficial vein, and Korean Application Publication No. 10-2020-0040097 on April 2, 2020 for an automatic body penetration device. These applications are hereby incorporated by reference in their entirety.

### [PRIOR ART DOCUMENTS]

Korean Patent No. 10-1601421 (Title of Invention: Automatic blood collection method, Publication date: March 10, 2016)

### DISCLOSURE OF THE INVENTION

### TECHNICAL PROBLEM

The purpose of the present invention is to provide an automatic invasive device for body, which includes a body press unit to more accurately sense a puncturing position. When the position of the body to be subjected to the invasive procedure is determined, the invasive procedure is performed by moving this invasive device to the corresponding position.

Another purpose of the present invention is to provide an automatic invasive device for body, which includes a probe unit that can rotate in the width direction of the body so as to more accurately sense a puncturing position.

Another purpose of the present invention is to provide an automatic invasive device for body, which includes a vacuum tube driving unit for automatically inserting a vacuum tube into a syringe of a syringe needle unit. When the position of the body to be subjected to the invasive procedure is determined, the invasive procedure is performed by moving this invasive device to the corresponding position.

Another purpose of the present invention is to provide an automatic invasive device for body, which includes a body contact material supply unit. For example, when the position of the body to be subjected to an invasive procedure is confirmed to perform the invasive procedure, the body contact material supply unit is used to attach a hemostatic band to a blood collection site after blood collection or to apply ultrasonic gel to the body prior to contact with an ultrasonic probe to sense the location of the blood collection.

### TECHNICAL SOLUTION

An automatic invasive device for body according to the present invention includes: a syringe needle unit support part configured to support a syringe needle that enters the body; and a press unit configured to press the body that has a site to be subjected to an invasive procedure.

The press unit may include: a press band that comes into contact with and presses the body to be pressed; at least one pair of moving units that support the press band; and a driving device that drives the moving units in a pressing direction and in a press releasing direction.

In an initial state prior to pressing, the press band may be provided not to come into contact with the body to be pressed.

The press unit may include: a guide bar; a first nut that moves along the guide bar in a first direction by the operation of the driving device; and a second nut that moves along the guide bar in a second direction opposite to the first direction, wherein the first nut and the second nut are connected to the at least one pair of moving units and move the moving units along the guide bar.

The automatic invasive device for body according to the present invention may further include a coupling that connects the guide bar to an output shaft of the driving device.

The automatic invasive device for body according to the present invention may further include: an ultrasonic probe that senses a puncturing position; and a probe unit that supports the ultrasonic probe and moves the ultrasonic probe in a certain direction.

A method for measuring blood pressure or pulse by using the automatic invasive device for body according to the present invention includes: a first operation of pressing the upper arm with the press unit; and a second operation of measuring the blood pressure or pulse as the probe unit moves the ultrasonic probe in a certain direction in the lower arm and measures a blood flow rate by using an ultrasonic image.

The method for measuring blood pressure or pulse according to the present invention may further include a first-first operation of changing pressure applied on the upper arm.

The automatic invasive device for body according to the present invention may further include a probe unit that includes a probe for sensing a human body part to be subjected to an invasive procedure, a probe support part, and a driving device. The probe unit may be rotated, by the operation of the driving device, in the width direction of the human body part to be subjected to the invasive procedure.

The automatic invasive device for body according to the present invention may further include a direction-changing part for changing the driving force of the driving device into a rotatory force that enables rotation of the probe unit in the width direction.

The probe unit may further include a pressing part for pressing the target body.

The probe unit may further include an elastic member that connects the pressing part to the probe support part.

The automatic invasive device for body according to the present invention may further include a linear driving device that moves the probe unit in the up-down direction.

The automatic invasive device for body according to the present invention may further include an insertion member-driving part that drives an insertion member to be inserted into a syringe needle unit. The insertion member-driving part may include: a tube support part that supports the insertion member; and a driving device that drives the insertion member support part.

The insertion member support part may include a gear tooth-shaped holder that comes into contact with and holds the insertion member.

The gear tooth-shaped holder may include a tooth portion that extends in the longitudinal direction of the insertion member support part.

The insertion member-driving part may include: a lead screw that receives the driving force of the driving device; a nut that is rotated by the rotation of the lead screw and moves along the lead screw; and a connection part which is connected to the nut and moved due to the movement of the nut, and is connected to the insertion member support part.

The insertion member-driving part may further include a guide that guides the connection part.

The gear tooth-shaped holder may rotate about a longitudinal axis of the insertion member support part.

The automatic invasive device for body according to the present invention may further include a body contact material supply unit. The body contact material supply unit may include: a roller unit that supplies a body contact material having a roll shape; and a press part that presses the body contact material so that the body contact material is brought into contact with the body at a certain position.

The roller unit may include: a first roller on which a body contact material roll before use is wound; a second roller on which a body contact material roll after use is wound; and a driving device that rotates at least one of the first roller or the second roller.

The roller unit may further include at least one switching roller which is provided between the first roller and the second roller and changes a direction so that the body contact material moves in a certain direction.

The switching roller may include: a first switching roller which is located on a downstream side of the first roller and changes a direction of a body contact material band supplied from the first roller; a second switching roller which is located on a downstream side of the first switching roller and changes the direction of the body contact material band supplied from the first switching roller; a third switching roller which is located on a downstream side of the second switching roller and changes the direction of the body contact material band supplied from the second switching roller; a fourth switching roller which is located on a downstream side of the third switching roller and changes the direction of the body contact material band supplied from the third switching roller; and a switching roller-driving device for driving at least one of the first to fourth switching rollers.

The second switching roller and the third switching roller may be arranged such that the body contact material band therebetween is placed above the body to be contacted.

### ADVANTAGEOUS EFFECTS

According to the present invention, there is an effect of providing the automatic invasive device for body, which includes the body press unit and is thus advantageous in sensing the puncturing position. In addition, the blood pressure or pulse can be measured together with the invasive procedure. Accordingly, the possibility of vasovagal syncope can be sensed in advance, and medical treatment therefor can be taken.

According to the present invention, the probe unit including the probe for sensing the puncturing position can be rotated in the width direction of the body. Accordingly, there is an effect of providing the automatic invasive device for body, which is advantageous in sensing the puncturing position.

According to the present invention, the vacuum tube driving device for automatically inserting the vacuum tube into the syringe of the syringe needle unit is provided. Accordingly, there is an effect of providing the automatic invasive device for body, which enhances convenience during the blood collection.

According to the present invention, the body contact material supply unit capable of attaching or applying the hemostatic band or ultrasonic gel to the body is provided. Accordingly, there is an effect of providing the automatic invasive device for body, which enhances convenience during the blood collection.

### BRIEF DESCRIPTION OF THE DRAWINGS

FIG. 1 is a block diagram of an automatic invasive device for body according to the present invention.
FIG. 2 is a front view of an incline adjustment unit of the automatic invasive device for body according to the present invention.
FIG. 3 is a side view of the incline adjustment unit of the automatic invasive device for body according to the present invention.
FIG. 4 is a side view of a conveyance unit of the automatic invasive device for body according to the present invention.
FIG. 5 is a plan view of the conveyance unit of the automatic invasive device for body according to the present invention.
FIG. 6 is a plan view of an incline adjustment unit according to another embodiment of the present invention.
FIG. 7 is a front view of a syringe needle unit operator of the automatic invasive device for body according to the present invention.
FIG. 8 is a left side view of the syringe needle unit operator of the automatic invasive device for body according to the present invention.
FIG. 9 is an enlarged front view of a vacuum tube driving unit according to the present invention.
FIG. 10 is an enlarged right side view of the vacuum tube driving unit according to the present invention.
FIG. 11 is a side view of a probe unit, a body press unit, and a body contact material supply unit of the automatic invasive device for body according to the present invention.
FIG. 12 is a side view of the probe unit according to the present invention and a view for explaining a method for sensing a puncturing position.
FIG. 13 is a front view of the probe unit, the body press unit, and the body contact material supply unit of the automatic invasive device for body according to the present invention.
FIG. 14 is an enlarged front view of the body press unit and the body contact material supply unit according to the present invention.
FIG. 15 is an enlarged side view of the body press unit and the body contact material supply unit according to the present invention.
FIG. 16 is a view illustrating an example of a body contact material band.
FIG. 17 is a flowchart of an automatic invasive method for body according to the present invention.
FIG. 18 is a flowchart of a target body sensing method in operations illustrated in FIG. 10.
FIGS. 19 to 22 are views for explaining a method for sensing a target blood vessel.

### MODE FOR CARRYING OUT THE INVENTION

Hereinafter, the present invention will be described in detail with reference to the accompanying drawings.

In this specification, only the minimum components necessary for the description of the present invention are described, and components not related to the essence of the present invention are not mentioned. Also, the present invention should not be construed in an exclusive sense that includes only the recited components, but should be interpreted in a non-exclusive sense to include other components not recited as well.

The exemplary embodiments described herein provide a general understanding of the principles of structures, functions, manufacture, use, and methods of a device disclosed herein. One or more embodiments are illustrated in the accompanying drawings. It will be understood by those skilled in the art that the device and method specifically described herein and illustrated in the accompanying drawings are non-limiting and exemplary embodiments and that the scope of the invention is defined by the appended claims. A feature shown and described in connection with one exemplary embodiment may also be combined with a feature of another embodiment. These modifications or variations are intended to be included within the scope of the present invention.

In the description of the present invention, the order of each operation should be understood to be non-limiting, unless the preceding operation must be performed logically and temporally before the subsequent operation. In other words, except for the exceptional case above, even if the process described in the subsequent operation is performed before the process described in the preceding operation, the nature of the invention is not affected, and the scope of the right should be defined regardless of the order of the operations. Also, "A or B" is defined herein to mean not only selectively referring to either A or B, but also including both A and B. In addition, the term "comprising" used herein has a meaning to encompass further including other components in addition to the components listed as being included.

The control method according to the present invention may be performed by electronic computing devices such as a computer, a tablet PC, a mobile phone, a portable computing device, and a stationary computing device. Also, it should be understood that one or more methods or aspects of the present invention may be performed by at least one processor. The processor may be installed in the computer, the tablet PC, the mobile device, the portable computing device, and the like. A memory configured to store computer program instructions may be installed in the above devices and specifically programmed to cause a processor to execute the program instructions in which programs are stored, thereby executing one or more processes as described herein. In addition, it should be understood that the information and method described herein may be performed by a computer, a tablet PC, a mobile device, a portable computing device, and the like that include one or more additional components and a processor. Also, the control logic may be implemented by a non-volatile computer readable medium including program instructions executable by a processor, a controller/control unit, or the like. Examples of computer readable media include, but not limited to, ROM, RAM, CD-ROM, magnetic tape, floppy disk, flash drive, smart card, optical data storage device, and the like. In addition, the computer readable recording medium may be distributed in computers connected to a network, and the computer readable medium may be stored and executed in a distributed manner, for example, by a remote server or a controller area network (CAN).

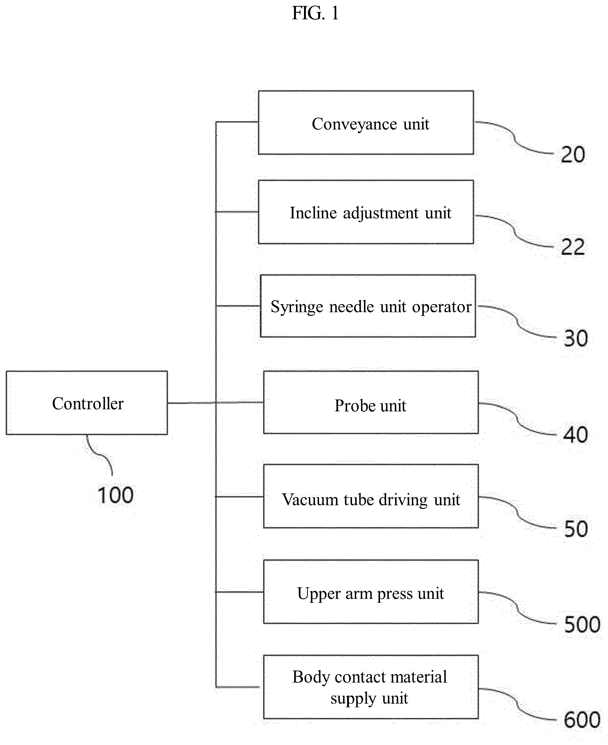

FIG. 1 illustrates a block diagram of an automatic invasive device for body according to the present invention. As illustrated in FIG. 1, the automatic invasive device for body according to the present invention include a controller 100, an incline adjustment unit 22, a conveyance unit 20, a syringe needle unit operator 30, a probe unit 40, a vacuum tube driving unit 50, a body press unit 500, and a body contact material supply unit 600.

The controller 100 controls the operations of the incline adjustment unit 22, the conveyance unit 20, the syringe needle unit operator 30, the probe unit 40, the vacuum tube driving unit 50, the body press unit 500, and the body contact material supply unit 600. The controller 100 may be a part of an electronic computing device, and refers to a logical combination of general-purpose hardware and software that performs the function thereof. The control command generated by the controller 100 for operating the automatic invasive device for body according to the present invention may be generated by a computer program stored in the aforementioned computer readable recording medium.

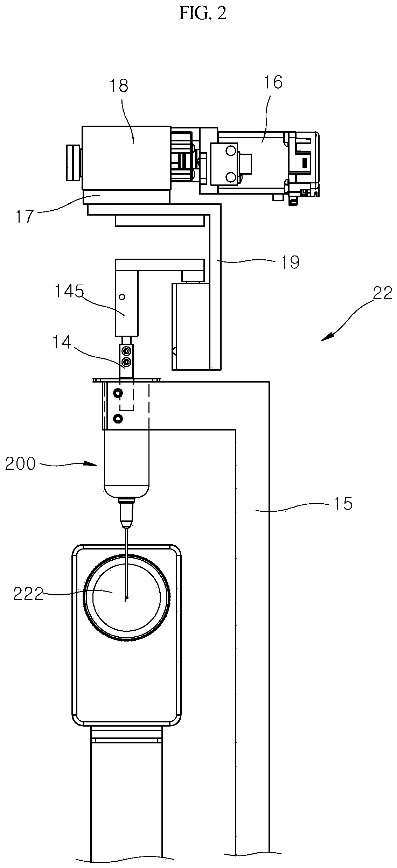

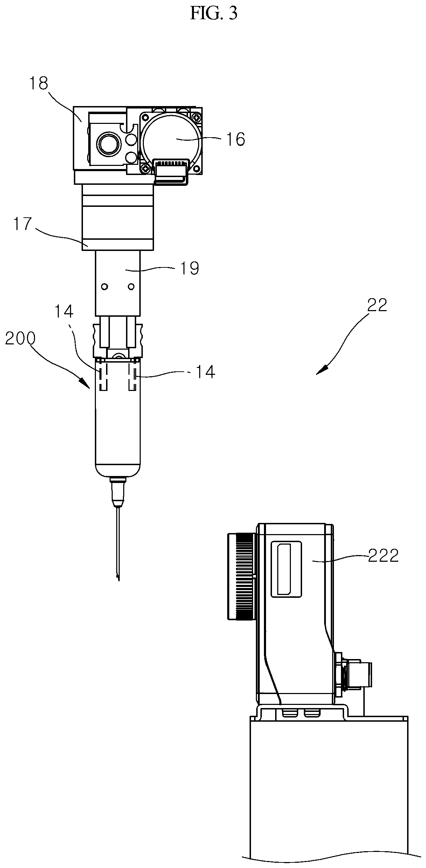

FIG. 2 illustrates a front view of the incline adjustment unit 22, and FIG. 3 illustrates a side view of the incline adjustment unit 22.

The incline adjustment unit 22 includes a first driving device 16, a first direction-changing part 18, a first rotary shaft 17, a body part 19, a second pneumatic device 145, a support arm 14, a support 15, and a detection part 222 (e.g., a vision sensor). The support arm 14 may be provided as a pair of arms parallel to each other as illustrated in FIG. 3, and may be driven in a direction toward or away from each other by the second pneumatic device 145. The support arm 14 moves from the inside of the syringe needle unit 200 in a direction away from each other and may support a syringe needle unit 200 while applying a force to the inner wall of the syringe needle unit 200 in an outward radial direction. The incline direction of an end of a syringe needle of the syringe needle unit 200 may be detected by the detection part 222.

When the detected incline direction of the syringe needle unit 200 provided on the support 15 is not a desired direction, the first driving device 16 is driven according to a control command of the controller 100 and rotates the body part 19 about the first rotary shaft 17. Accordingly, the incline direction of the syringe needle of the syringe needle unit 200 supported by the support arm 14 may be adjusted to the desired direction. The driving force of the first driving device 16 is changed into a direction for rotating the body part 19 by the first direction-changing part 18. The changing of the transmission direction of the driving force of the first driving device 16 may be made by employing various known kinematic structures, and the first direction-changing part 18 may include a speed reducer. A direction-changing part described herein later may also include a speed reducer if necessary. The first driving device 16 may include a rotary driving device, for example, a general motor, or a linear driving device, for example, a linear motor. When the linear motor is used, the first direction-changing part 18 changes the linear motion direction into a rotation direction for rotating the support arm 14.

A direction-changing part used in another component to be described later as well as the incline adjustment unit 22 is not an essential component and may not be used depending on the design of the driving device.

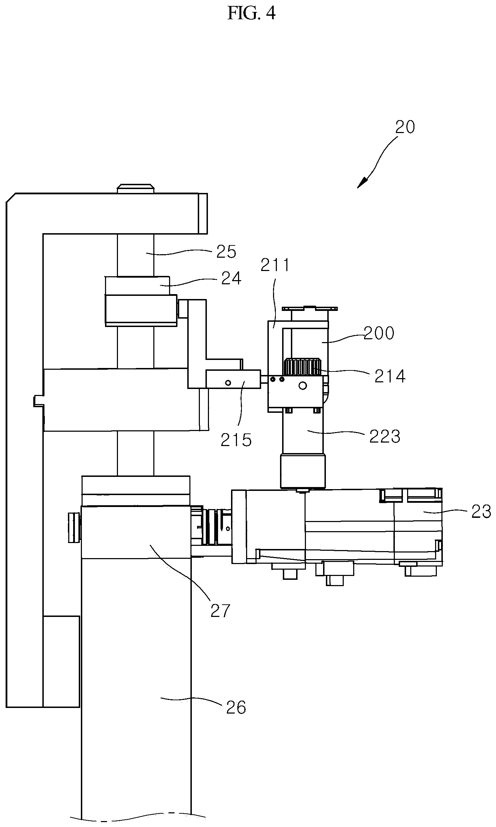

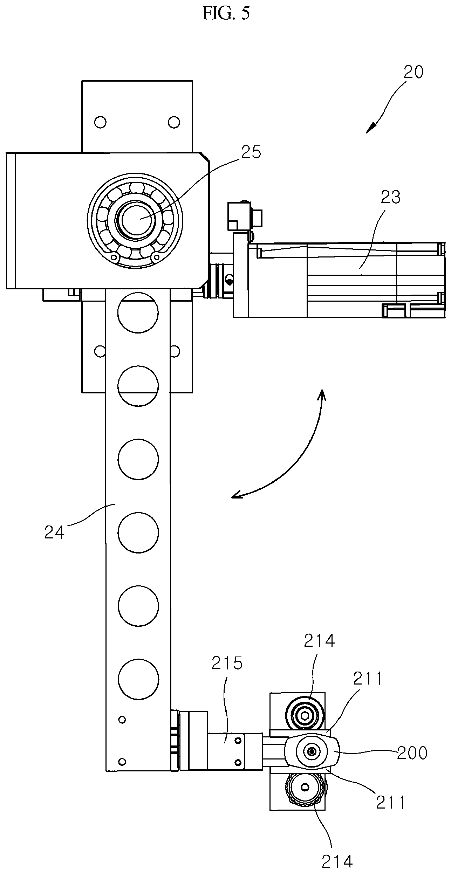

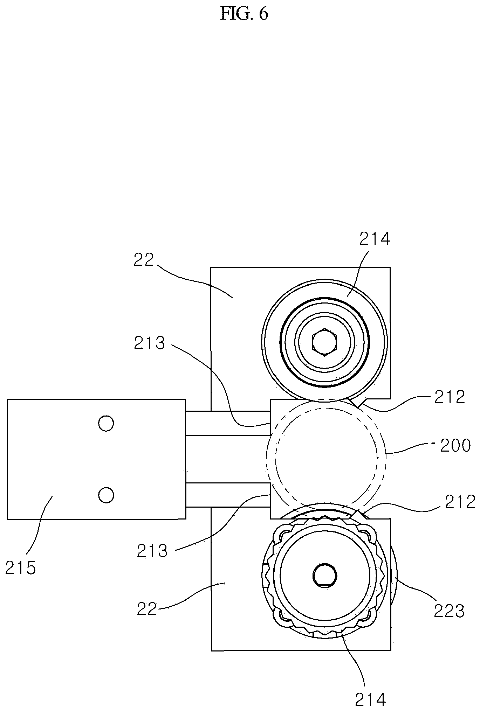

FIGS. 4 and 5 respectively illustrate a side view and a plan view of the conveyance unit 20 of the automatic invasive device for body according to the present invention. An incline adjustment unit according to another embodiment of the present invention may be provided in the conveyance unit 20 illustrated in each of FIGS. 4 and 5. A plan view of an incline adjustment unit according to another embodiment of the present invention is illustrated in FIG. 6.

The conveyance unit 20 includes a second driving device 23, a movable arm 24, a second rotary shaft 25, a first body part 26, and a second direction-changing part 27. An incline adjustment unit according to another embodiment of the present invention may be provided in the movable arm 24. This incline adjustment unit may include at least one roller 214 in contact with the syringe needle unit 200, a seventh driving device 223 that rotates the roller 214 to rotate the syringe needle unit 200, and a detection part 222 (not illustrated in FIGS. 4 to 6) . The syringe needle unit 200 may maintain a certain position as the outside thereof is supported by a first holder 211. The first holder 211 may include at least a pair of arms parallel to each other, and the arms may be driven in a direction toward or away from each other, for example, by a third pneumatic device 215. When the holder 211 comes close to each other and comes into contact with the outside of the syringe needle unit, the holder may support the syringe needle unit by applying a force in a direction toward the inside of the syringe needle unit 200. The holder 211 may support the syringe needle unit 200 from the inside thereof like the support arm 14.

A tooth portion is provided on the outer circumferential surface of the roller 214 and may be in contact with the outer circumferential surface of the syringe needle unit 200. When the outer circumferential surface of the syringe needle unit 200 is provided with a tooth portion corresponding to the tooth portion, these tooth portions may come into contact and engage with each other. The material of the roller 214 may be silicone, synthetic resin, or metal.

The incline adjustment unit illustrated in FIG. 6 may further include movement limiting parts 212 and 213 that prevent the syringe needle unit 200 from moving in a direction other than the rotation direction of the syringe needle unit 200 dependent on the rotation of the roller 214. A plurality of movement limiting parts 212 and 213 may be provided on the outer circumference of the syringe needle unit 200.

The movable arm 24 may be moved between the first position and the second position by the driving of the second driving device 23. As used herein, the term "first position" refers to a position at which the syringe needle unit 200 is provided to the conveyance unit 20, and the term "second position" refers to a position at which the syringe needle unit 200 is delivered from the conveyance unit 20 to the syringe needle unit operator 30. The incline adjustment unit 22 may be adjacent to the first position or provided at the first position, or may be adjacent to the second position or provided at the second position. Alternatively, the incline adjustment unit may be also provided at a third position other than the first position and the second position. In such a case, the movable arm 24 may convey the syringe needle unit 200 from the first position to the third position so that the incline direction is adjusted at the third position, and may convey the syringe needle unit 200 from the third position to the second position. In this embodiment, the movable arm for conveying the syringe needle unit 200 from the first position to the third position may be configured separately from the movable arm for conveying the syringe needle unit 200 from the third position to the second position.

The position of the incline adjustment unit 22 is independent of the essential technical idea of the present invention. Also, a plurality of incline adjustment units may be provided. When the incline adjustment unit is provided at the second position, the movable arm 24 may not be necessary.

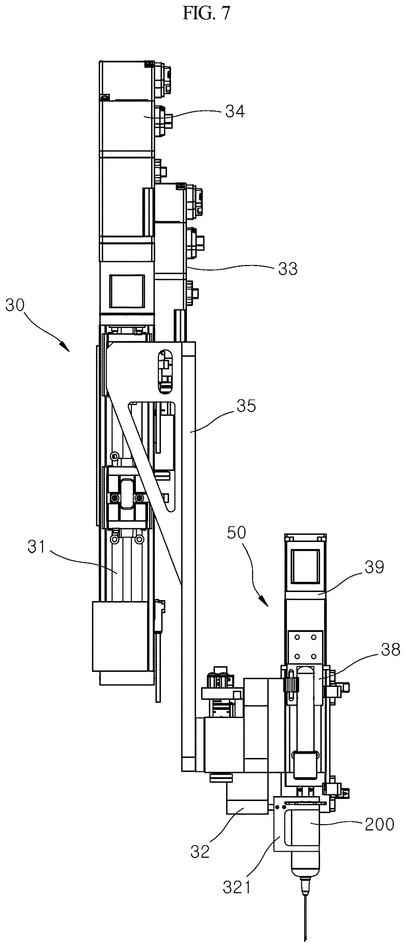

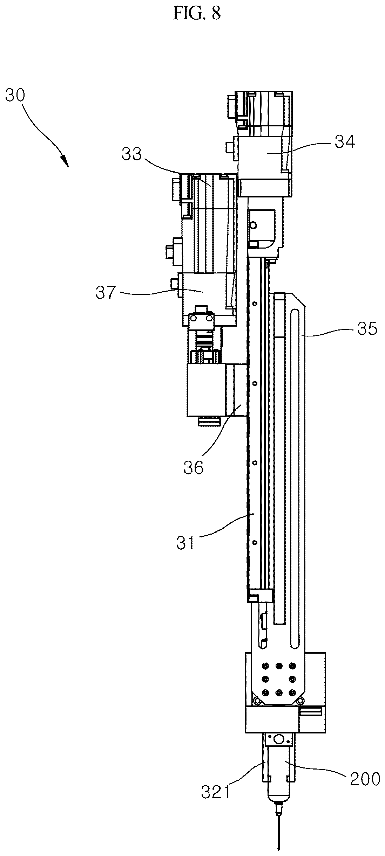

FIG. 7 illustrates a front view of the syringe needle unit operator 30 of the automatic invasive device for body according to the present invention, and FIG. 8 illustrates a left side view thereof.

The syringe needle unit operator 30 includes a second body part 31, a first pneumatic device 32, a third driving device 33, a fourth driving device 34, a connection member 35, a third rotary shaft 36, a third direction-changing part 37, and a vacuum tube driving unit 50.

The syringe needle unit 200 is supported on the connection member 35, and this syringe needle unit may be supported by at least a pair of second holders 321 driven by the first pneumatic device 32. Also, the vacuum tube driving unit 50 may be supported on the connection member 35. A vacuum tube (not shown) is a device that is inserted into the syringe needle unit 200 and can suction the blood during blood collection. In this specification, the vacuum tube inserted into the syringe needle unit 200 is described for convenience of description, but driving other components that can be inserted into the syringe needle unit is also included in the present invention.

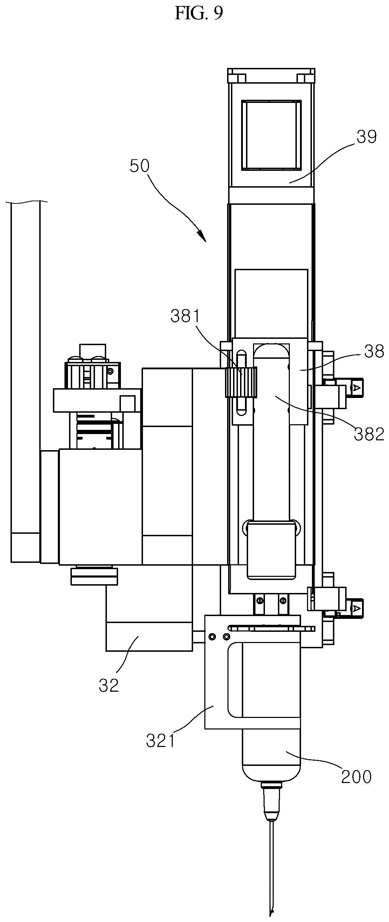

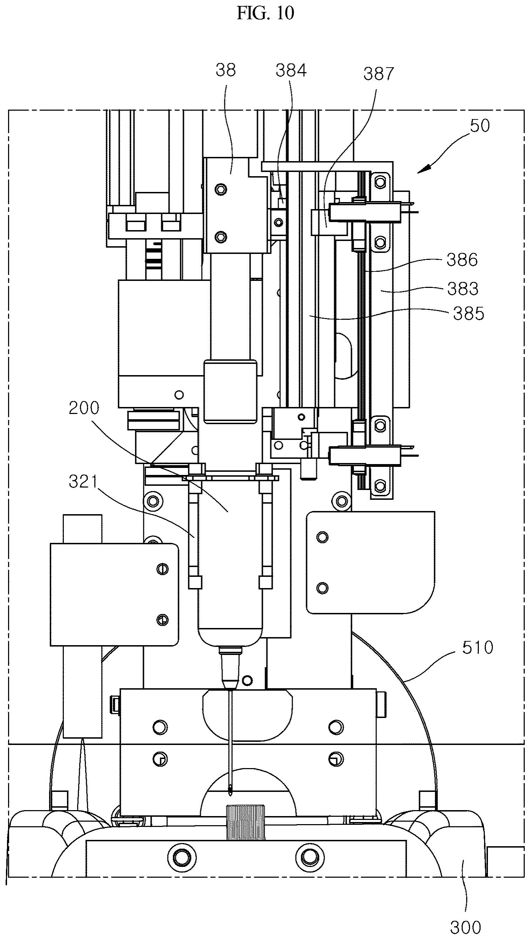

FIG. 9 illustrates an enlarged front view of the vacuum tube driving unit 50, and FIG. 10 illustrates an enlarged right side view of the vacuum tube driving unit 50.

The vacuum tube driving unit 50 includes a bracket 383 connected to the connection member 35, a vacuum tube support part 38, a lead screw 385, a nut 384, a connection part 387, and an eighth driving device 39. A guide 386 is provided in the longitudinal direction of the bracket 383. The connection part 387 is coupled to the nut 384 so as to move along with the movement of the nut 384.

The connection part 387 connects the vacuum tube support part 38 and the guide 386.

A recess 382 (see FIG. 9), into which a vacuum tube (not shown) is inserted, is provided in the vacuum tube support part 38, and a gear tooth-shaped holder 381 that contacts the side surface of the vacuum tube to be inserted may be provided in the recess 382. A tooth portion, which extends in the longitudinal direction of the vacuum tube support part 38, may be provided in the gear tooth-shaped holder 381. This gear tooth-shaped holder 381 may facilitate the insertion of the vacuum tube and prevent the vacuum tube from being separated. Also, as the tooth portion extending in the longitudinal direction of the vacuum tube support part 38 is provided, the straightness and center of the vacuum tube may be maintained when the vacuum tube is inserted into the syringe needle unit 200. The gear tooth-shaped holder 381 may rotate about the longitudinal axis of the vacuum tube support part 38.

When the movable arm 24 moves from the first position to the second position, the second holders 321 support the syringe needle unit 200 by the operation of the first pneumatic device 32 at the second position. Subsequently, as the holding state between the holder 211 and the syringe needle unit 200 is released by the operation of the third pneumatic device 215, the syringe needle unit 200 may be delivered. After the syringe needle unit 200 is delivered, the movable arm 24 may return to the first position.

The connection member 35 may be provided with an incline adjustment unit having a configuration as illustrated in FIGS. 4 to 6. As illustrated in FIG. 7, the syringe needle unit 200 is supplied to the syringe needle unit operator 30, and an incline adjustment unit is provided in the syringe needle unit operator 30. In this embodiment, the conveyance unit 20 may not be necessary.

In a state in which the syringe needle unit 200 is delivered to the syringe needle unit operator 30 and supported by the second holders 321, the vacuum tube fitted to the vacuum tube support part 38 may be inserted into the syringe needle unit 200. The vacuum tube may be supported by the vacuum tube support part 38 in advance, or the vacuum tube may be inserted into the vacuum tube support part 38 in a state in which the syringe needle unit 200 is supported by the second holders 321.

In a state in which the vacuum tube is fitted to the vacuum tube support part 38, the nut 384 moves downward along the lead screw 385 when the eighth driving device 39 is operated. The connection part 387 moves together by the movement of the nut 384, and the vacuum tube support part 38 connected to the connection part 387 is moved toward the syringe needle unit 200 along the guide 386 and inserted into the syringe needle unit 200.

The syringe needle unit 200, into which the vacuum tube has been inserted, is aligned in an orientation enabling invasive procedure into the body by the third driving device 33 and the fourth driving device 34, thereby performing the invasive procedure into the target body.

First, the third driving device 33 is driven to rotate the second body part 31 about the third rotary shaft 36 so as to obtain a certain angle of orientation enabling the invasive procedure. The driving force of the third driving device 33 is changed into the rotatory force about the third rotary shaft 36 by the third direction-changing part 37. In a state in which the second body part 31 is rotated at a certain angle, the fourth driving device 34 is driven to move the connection member 35 along the longitudinal direction of the second body part 31, thereby performing the invasive procedure. A ball screw is provided along the longitudinal direction of the second body part 31 so that the connection member 35 can move.

The adjustment of the orientation angle of the syringe needle unit 200 to enable the invasive procedure by driving the third driving device 33 may be performed at a separate third position, and then the syringe needle unit 200 may be conveyed to the position enabling the invasive procedure.

Although not illustrated in FIGS. 7 and 8, the second body part 31 may move in at least one or the other of the left-right direction of FIG. 7 or the left-right direction of FIG. 8.

The second body part 31 may be adjusted to be in an orientation enabling the invasive procedure by the third driving device 33, but the orientation may be manually adjusted without the third driving device 33. For example, the orientation may be adjusted as the second body part 31 is rotated by a user so as to form a certain angle with respect to the body to be subjected to the invasive procedure.

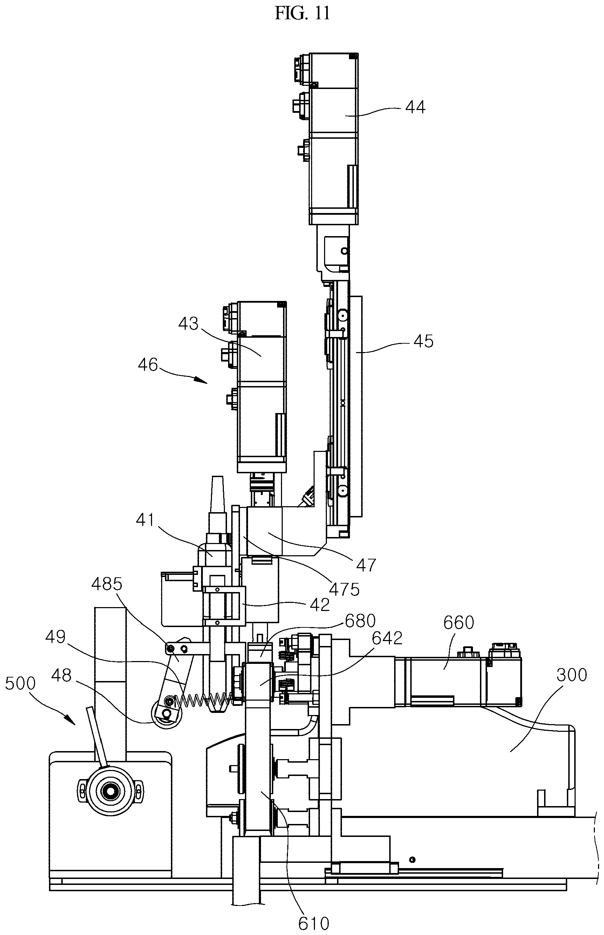

FIG. 11 illustrates a side view of the probe unit 40, the body press unit 500, and the body contact material supply unit 600.

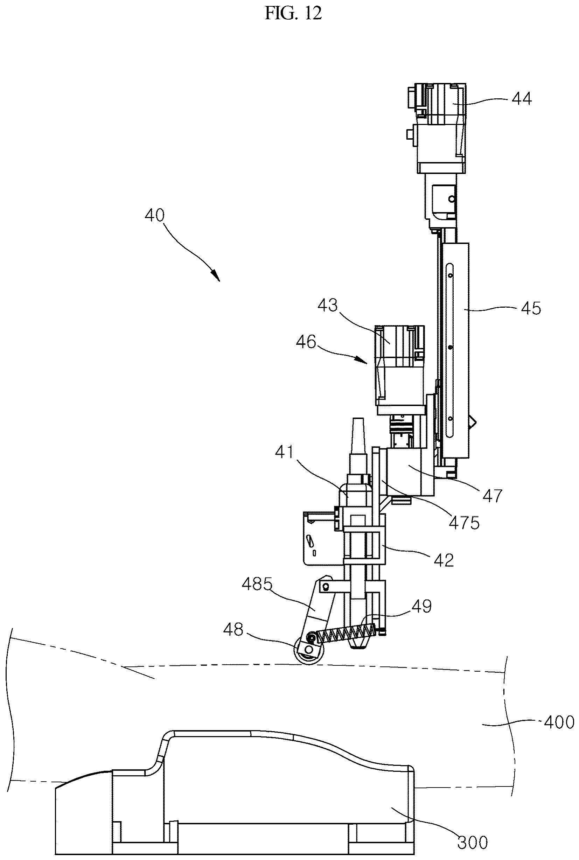

The probe unit 40 according to the present invention includes a probe 41, a probe support part 42, a fifth driving device 43, a sixth driving device 44, a rail part 45, a movable part 46, a fourth direction-changing part 47, a pressing part 48 for pressing the target body, and a pressing part holder 485. The probe 41 may include, for example, an ultrasonic probe. The pressing part 48 may be rotatably supported by the pressing part holder 485 and have a roller shape. The pressing part 48 and the probe support part 42 may be connected to each other by an elastic member 49 (e.g., a spring) so that the pressing part 48 presses the body at a certain force. FIG. 12 illustrates a side view of the probe unit 40 for explaining a state in which the pressing part 48 presses the body. In order to clearly illustrate the above state, the illustrations of the body press unit 500 and the body contact material supply unit 600 are omitted.

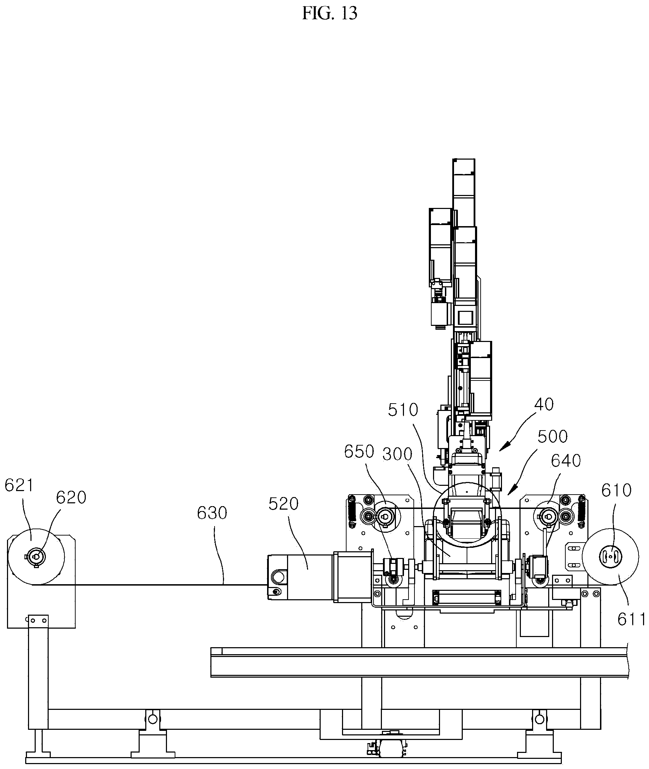

The probe support part 42 may be rotated about a fourth rotary shaft 475, that is, may be rotated in the left-right direction when viewed from the front side illustrated in FIG. 13, by the operation of the fifth driving device 43.

When the probe support part 42 rotates about the fourth rotary shaft 475, the probe 41 supported by the probe support part 42 moves in the width direction of the human body part supported by the body support part 300, for example, the lower arm while sensing a puncturing position.

The driving force of the fifth driving device 43 is changed into the rotatory force for rotating the probe support part 42 about the fourth rotary shaft 475 by the fourth direction-changing part 47.

The movable part 46 may move along the longitudinal direction of the rail part 45 by the operation of the sixth driving device 44. The rail part 45 may be provided with a ball screw.

The probe unit 40 may move along the longitudinal direction of a body 400. An example of a method for sensing the position of a target body (e.g., a superficial vein) will be described later. When the target body is a blood vessel, the position is easily sensed after straightening. Accordingly, while the probe unit 40 is moved along the longitudinal direction of the body 400 (e.g., a lower arm) placed on a body support part 300, the pressing part 48 may press and straighten the body. Also, the range in which the pressing part 48 presses the target body for straightening is a meaningful distance for sensing same, and this may be set within a predetermined range.

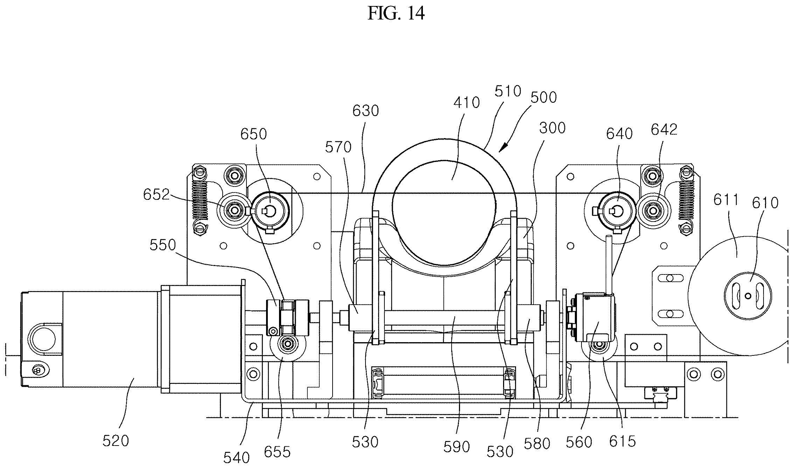

The body press unit 500 is provided in front of the probe unit 40. The body press unit 500 according to the present invention will be described with reference to FIGS. 13 and 14. In this specification, the case where the human body part to be pressed is the upper arm is described for convenience of description, but the human body part to be pressed may be a part other than the upper arm.

The body press unit 500 includes a press band 510, a ninth driving device 520, at least a pair of moving units 530 for supporting both sides of the press band 510, a first support frame 540, a coupling 550, an encoder 560, a first nut 570, a second nut 580, and a guide bar 590 on which the moving units 530 is supported and which guides the movement of each moving unit. The guide bar 590 may include a lead screw. The first nut 570 and the second nut 580 are connected to the at least one pair of moving units 530, respectively, and the at least one pair of moving units 530 may move together according to the movements of the first nut 570 and the second nut 580.

When a lower arm 400 is placed on the body support part 300, an upper arm 410 passes through the press band 510. In an initial state prior to pressing, the press band 510 may be provided not to come into contact with the upper arm 410. When the press band 510 does not contact the upper arm 410 before performing pressing, it is possible to prevent the skin from being rubbed by the press band during pressing and getting abrasions.

The coupling 550 transmits the driving force of the ninth driving device 520 by connecting an output shaft of the ninth driving device 520 to the guide bar 590. The first nut 570 and the second nut 580 are moved along the guide bar 590 by the operation of the ninth driving device 520, and the first nut 570 and the second nut 580 have screw threads formed such that these nuts move in opposite directions along the guide bar 590.

In FIG. 14, the first nut 570 moves together with the left moving unit 530, and the second nut 580 moves together with the right moving unit 530.

The encoder 560 is a sensor for sensing the rotation speed and direction of the ninth driving device 520.

An upper arm press unit 500 may further include a pressure sensor (not shown) capable of measuring pressure applied to the upper arm.

Next, the body contact material supply unit 600 will be described.

The body contact material may be a hemostatic band attached to the blood collection site for hemostasis after blood collection, or may be a gel applied to the skin before blood collection so that the ultrasonic probe senses the location of the blood collection.

According to the present invention, while the body contact material is continuously supplied in the form of a roller, this body contact material may be accurately provided at a puncturing position on the body or a desired position.

The body contact material supply unit 600 includes a first roller 610, a first switching roller 615, a second switching roller 640, a third switching roller 650, a fourth switching roller 655, and a second roller 620. A first band roll 611 is wound on the first roller 610, and a second band roll 621 is wound on the second roller 620. When the first band roll 611 is a body contact material roll before use, the second band roll 621 is a body contact material roll after use, that is, a roll from which the body contact material has been removed. When the second band roll 621 is a body contact material roll before use, the first band roll 611 is a body contact material roll after use, that is, a roll from which the body contact material has been removed.

When the first band roll 611 is the body contact material roll before use, the first band roll 611 moves from the first roller 610 to the second roller 620, via the first switching roller 615, the second switching roller 640, the third switching roller 650, and the fourth switching roller 655. Then, the body contact material roll after use is wound on the second roller 620.

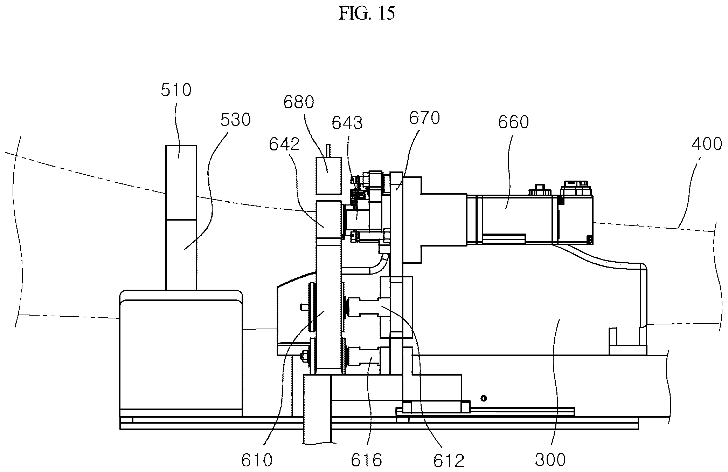

The second switching roller 640 is driven by a tenth driving device 660 (see FIG. 15), and the second roller 620 is driven by an eleventh driving device (not shown). Accordingly, a band 630 may be supplied in the direction described above.

A first auxiliary roller 642 moves the band in the supply direction in conjunction with the second switching roller 640. A second auxiliary roller 652 moves the band in the supply direction in conjunction with the third switching roller 650.

As illustrated in FIG. 15, the band 630 may be disposed to pass over the lower arm 400, and a press part 680, which presses the band 630 to attach or apply the body contact material attached to the band 630 to the body, is disposed above the band 630. The press part 680 may be driven by a certain driving means, for example, a solenoid valve (not shown) to press the upper portion of the band 630 at a certain position.

The body contact material supply unit 600 according to the present invention may control the position in a plurality of directions along a certain rail system, and may be disposed at a position for attaching or applying the body contact material. For example, the position control may be performed by establishing a connection with a rail system that can move, in a plurality of directions, a certain bracket for supporting the above-described body contact material supply unit 600. The technique for position control by moving the bracket in a plurality of directions through the rail system can be easily implemented by applying a known technology. Also, this technique is not an essential technical idea of the present invention, and thus, a detailed description thereof will be omitted.

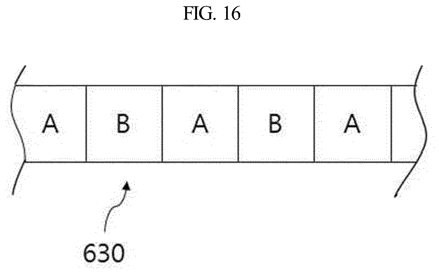

FIG. 16 illustrates one example of the band 630 according to the present invention. The band 630 may be provided with a body contact material, for example, a hemostatic band for stopping bleeding after blood collection or a gel for sensing the puncturing position using an ultrasonic probe. The gel provided to the band 630 may be in the form of a solid gel.

When the hemostatic band and the solid gel are provided in the band 630, first sections A and second sections B may be alternately arranged. For example, hemostatic bands may be located in the first sections A, and solid gels may be located in the second sections B. The number of body contact materials provided to the band 630 is not limited, and two or more types of contact materials may be provided as needed.

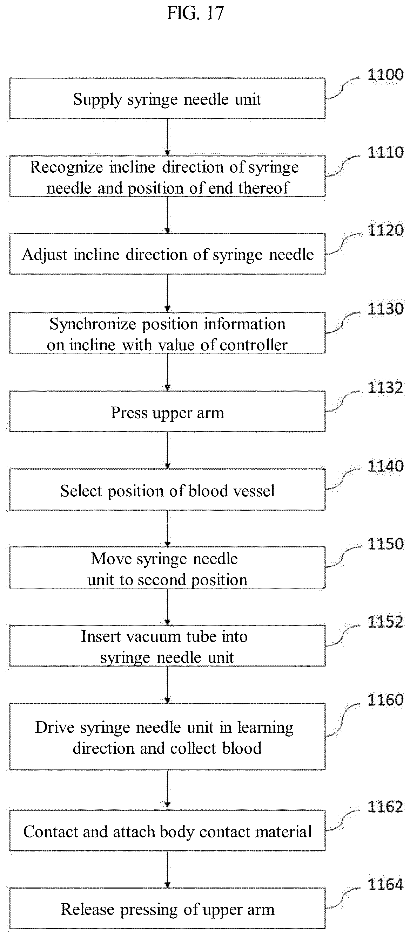

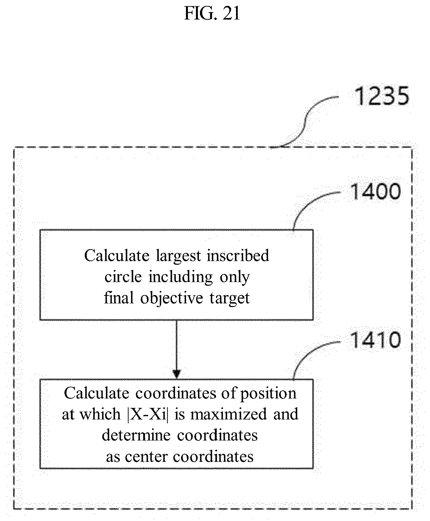

Next, an operation and control method of the present invention will be described. The order of each operation in FIGS. 17 and 21 should be understood to be non-limiting, unless the preceding operation must be performed logically and temporally before the subsequent operation. In other words, except for the exceptional case above, even if the process described in the subsequent operation is performed before the process described in the preceding operation, the nature of the invention is not affected, and the scope of the right should be defined regardless of the order of the operations.

In an operation (1100), the syringe needle unit 200 is supplied. The syringe needle unit 200 may be supplied to the incline adjustment unit 22. In the embodiment illustrated in FIGS. 2 and 3, the syringe needle unit 200 may be supplied to the support 15 and supported thereby. In the embodiment of the incline adjustment unit illustrated in FIGS. 4 to 6, the syringe needle unit 200 may be supplied such that the syringe needle unit 200 is supported by the holder 211 and located between the rollers 214. As described above, the incline adjustment unit may be provided at or adjacent to at least one of a first position or a second position, and may be provided at or adjacent to a third position other than the first position or the second position.

When the syringe needle unit 200 is supplied, the detection part 222 recognizes the direction of the incline of the syringe needle and the position of an end thereof (operation (1110)). When the recognized incline direction of the syringe needle is different from the set direction, the first driving device 16 or the seventh driving device 223 is operated by receiving a command from the controller 100. Accordingly, the incline direction of the syringe needle is adjusted by rotating the syringe needle unit 200 (operation (1120)). When the first driving device 16 operates, the body part 19 rotates about the first rotary shaft 17, and the syringe needle unit 200 supported by the support arm 14 rotates.

In the incline adjustment unit according to another embodiment of the present invention illustrated in FIGS. 4 to 6, the roller 214 rotates when the seventh driving device 223 operates. Accordingly, the incline direction is adjusted as the syringe needle unit 200 rotates.

When the incline direction of the syringe needle adjusted by the detection part 222 reaches a set value, the first driving device 16 or the seventh driving device 223 stops operating, and the position information of the adjusted incline is synchronized with the value recorded in the controller 100 (operation (1130)).

In an operation (1132), the body press unit 500 presses the upper arm. When the ninth driving device 520 operates, the guide bar 590, which is connected to the output shaft of the ninth driving device 520 via the coupling 550, rotates. Due to this rotation, the first nut 570 and the second nut 580 move in a direction closer to each other, and the press band presses the upper arm 410.

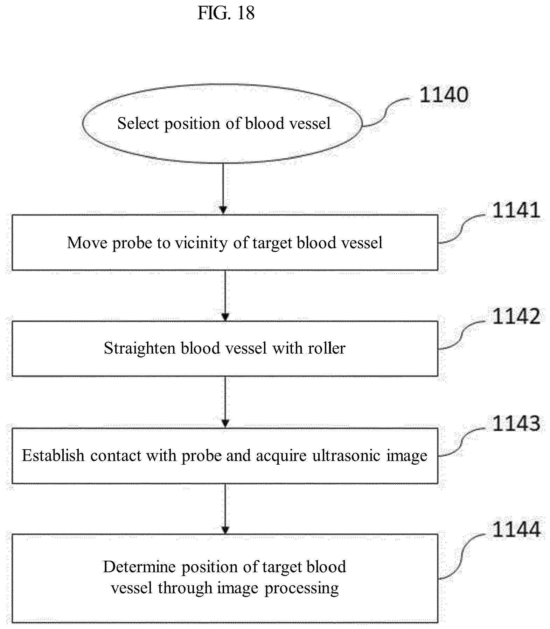

Next, the position of the target body is selected (operation (1140)). The selection process when the target body is a superficial vein is illustrated in FIG. 18.

First, the probe 41 is moved near the blood vessel (operation (1141)). Subsequently, the probe unit 40 is moved while the blood vessel is pressed by the pressing part 48, and thus, the blood vessel is straightened (operation (1142)). The pressing part 48 may be allowed to move only by a certain range on the target body. When the blood vessel is straightened by the pressing part 48, ultrasonic scanning and blood collecting are facilitated. The force that the pressing part 48 presses against the blood vessel is a constant force optimized in the range of 0 to 19.6 N.

When the straightening of blood vessel is completed, the probe 41 is brought into contact with the target body, an ultrasonic image is acquired (operation (1143)), and the position of the target blood vessel is determined through image processing of the acquired ultrasonic image (operation (1144)).

Meanwhile, at the same time as determining the position of the target blood vessel or with a time gap therebetween, the blood pressure and pulse may be measured using the ultrasonic probe 41. That is, when the ultrasonic probe 41 is brought into contact with the body so as to sense the position of the blood vessel, the blood pressure and pulse may be measured together by measuring the blood flow rate. When blood pressure and/or pulse are measured together, the possibility of vasovagal syncope, which may occur during blood collection, can be checked and handled in advance.

When the blood pressure or pulse is measured, the upper arm press unit 500 changes the pressure applied to the upper arm, and the blood flow rate may be measured through an image acquired by moving the ultrasonic probe 41. The blood flow rate may be measured by moving the ultrasonic probe 41 in a direction toward or away from the upper arm.

In an operation (1150), the syringe needle unit 200 is moved to the second position. When the incline adjustment unit 22 is located at the first position or adjacent to the first position, in a state in which the movable arm 24 supports the syringe needle unit 200 of which the incline direction has been adjusted, the second driving device 23 is operated by receiving a command from the controller 100 and rotates the movable arm 24 about the second rotary shaft. Accordingly, the movement from the first position to the second position becomes possible. The movement from the first position to the second position does not necessarily have to be made only through the rotation of the movable arm 24. Any structure may be applied as long as the structure is a kinematic position-changing structure that is known or obvious to those skilled in the art from known structures.

When the incline adjustment unit 22 is disposed at or adjacent to the second position, the operation (1150) may be unnecessary. In this embodiment, in a state in which the syringe needle unit 200 is supported by the syringe needle unit operator 30, the syringe needle unit illustrated in FIGS. 2 and 3 or FIGS. 4 to 6 adjusts the incline direction.

When the incline adjustment unit 22 is provided at the third position, the movable arm 24 may move from the first position to the third position, and may move to the second position after adjusting the incline direction at the third position.

After the syringe needle unit 200 is moved to the second position or after the incline adjustment is performed at a position adjacent to the second position, the vacuum tube is inserted into the syringe needle unit 200 (operation (1152)).

When the eighth driving device 39 operates, the lead screw 385 rotates. Accordingly, the nut 384 moves along the lead screw 385. The connection part 387 moves along the guide 386 by the movement of the nut 384, and the vacuum tube support part 38 connected to the connection part 387 moves in the direction toward the syringe needle unit 200 and is then inserted therein.

In a state in which the syringe needle unit 200, into which the vacuum tube has been inserted, is supported by the syringe needle unit operator 30, the third driving device 33 operates according to the command of the controller 100, and rotates the second body part 31 to a certain angle, that is, to an angle for invasive procedure into the body. The fourth driving device 34 receives a command from the controller 100 to move the connection member 35 in the longitudinal direction of the second body part 31, and accordingly, the invasive procedure into the body is performed (operation (1160)). When the target body is a superficial vein, the blood collection may be performed after the invasive procedure. Although not illustrated in FIGS. 7 and 8, the second body part 31 may move in at least one or the other of the left-right direction of FIG. 7 or the left-right direction of FIG. 8.

When the blood collection is completed, a blood collection band may be attached to the blood collection site, that is, the puncturing position (operation (1162)). The press part 680 is moved downward by a driving device (e.g., a solenoid valve, not shown), and the band 630 is pressed to come into contact with the blood collection site. Accordingly, the hemostasis band provided in the band 630 may be attached to the blood collection site. When the contact/attachment is completed, the press part 680 moves upward. Subsequently, the second switching roller 640 and the third switching roller 650 are driven so that a next body contact material comes to the corresponding position.

When the body contact material is a solid gel, the operation (1162) may be performed before the operation (1140).

In an operation (1164), the pressed state of the upper arm is released. When the ninth driving device 520 is operated so that the first nut 570 and the second nut 580 move in directions away from each other, the moving units 530 move in directions away from each other, and thus, the pressed state of the press band 510 pressing the upper arm 410 is released.

Hereinafter, an example of a method for sensing the position of a body part (a superficial vein is described as an example) to be subjected to the invasive procedure by processing an image acquired by the ultrasonic probe 41 will be described. The sensing method described below relates to the content disclosed in Korean Application Publication No. 10-2020-0010302 of the present applicant. This is meaningful only for illustrative purposes described to aid understanding of the present invention and does not limit the scope of the present invention.

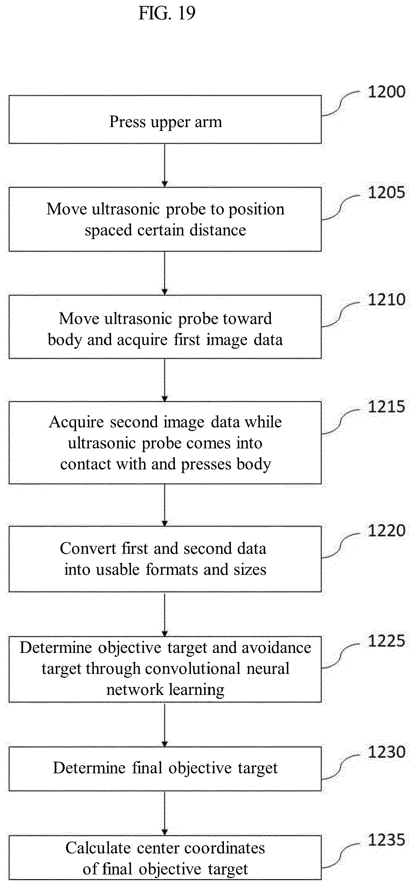

FIG. 19 illustrates a flowchart of a position determination method for a superficial vein through probe sensing.

In order to search for the position of the superficial vein, a subject first puts the upper arm in an upper arm compression unit (not shown) and performs upper arm compression (operation (1200)). The upper arm compression unit includes a pressing means of 1 to 5 cm, and the pressing means presses the upper arm at 3 to 12 cm above the elbow, preferably 6 to 8 cm. The pressing pressure may be between 10 and 60 mmHg, preferably between 20 and 30 mmHg. The process described below may be performed without the upper arm compression unit compressing the upper arm.

FIG. 19 illustrates that the upper arm compression is performed before the ultrasonic probe 41 is moved. However, the ultrasonic probe 41 is moved, the ultrasonic probe 41 starts to acquire first image data, and then, the upper arm compression may be performed. When the upper arm compression is performed after the acquisition of the first image data is started, it is possible to obtain both the first image data before the upper arm compression is performed and the first image data after the upper arm compression is performed.

Referring back to FIG. 19, the ultrasonic probe 41 is moved to a position spaced apart a certain distance, for example, 1 mm from the body of the subject after the upper arm compression (operation (1205)).

The position of the body of the subject, at which the ultrasonic probe 41 is located, may be 0 to 8 cm, preferably 1 to 3 cm from the bend of the arm, at which the median cubital vein is positioned, in a direction toward the hand.

In an operation (1210), first image data is acquired while the ultrasonic probe 41 is moved toward the body of the subject. The first image data is image data before the ultrasonic probe 41 comes into contact with the body of the subject, and may include both image data before the upper arm is pressed and image data after the upper arm is pressed.

Second image data is collected while the ultrasonic probe 41 is in contact with and presses the body of the subject. According to another embodiment of the present invention, the body of the subject may be pressed by another means other than the ultrasonic probe 41.

The first and second image data may include image data analyzed through the Doppler effect among sound wave signals of blood flow. According to another embodiment of the present invention, only one of the first image data or the second image data may be used.

The obtained first and second image data are converted, by an image data processing module 30, into formats and sizes that can be used by the program that executes the method according to the present invention (operation (1220)). According to another embodiment of the present invention, image data analysis, which will be described later, may be performed without converting the image data.

The converted image data is analyzed by a machine learning server 140, for example, a convolutional neural network, pixels of an objective target and an avoidance target are displayed in the image data (operation (1225)).

As used herein, the objective target refers to blood vessels such as superficial veins or arteries, and the avoidance target refers to arteries, nerves, bone tissue, and the like. The objective target and the avoidance target may be determined according to the use. For example, depending on the use, arteries, nerves, bone tissue, and the like determined as the avoidance target when checking the position of the superficial vein may be rather set as the objective target.

As a method for displaying the objective target and the avoidance target, these targets may be displayed in units of pixels, and may also be displayed as a bound-box through a median value, a boundary, and a distance. Also, the median value, the information on a shape such as a circle/ellipse and a rectangle, and the information capable of specifying a shape such as a radius/distance information of two or more points/length of one side may be stored together.

The convolutional neural network learning, which is an example of machine learning to be used in the present invention, will be described.

For the convolutional neural network learning, it is necessary to perform learning through training data in advance. The image data used for the convolutional neural network learning may be preprocessed to increase generality of the data by a data augmentation technique that includes random cropping, size transformation, horizontal flip, and the like of the first and second image data as described above.

The machine learning server acquires and stores information that matches the characteristics of the objective target and the avoidance target through the convolutional neural network learning.

This characteristic information may include at least one of: the intensity of echogenicity of components identified in ultrasonic image data; the pattern information where echogenic components are distributed; the relative position information where the components identified in the image data are distributed; the information about the height, weight, gender, and age of a subject, a contracted disease, and past or current treatment; the information when pressed by an ultrasonic probe or other means; or the real-time flow information of blood flow analyzed through the Doppler image.

When the skin surface is pressed with an ultrasonic probe or other means, veins are pressed directly and reduced in size unlike arteries. Also, proximal portions of veins are pressed to cause blood congestion, thereby inflating. The information when pressed may include this information.

In the case of an artery, which is one of the avoidance targets, blood flow is fast unlike a vein, and thus, the Doppler signal is strong and pulsates while periodically increasing and decreasing. Therefore, the real-time information of blood flow is useful for sensing a target.

The convolutional neural network learning may be trained to display pixels that correspond to the positions of the objective target and the avoidance target in the image data on the basis of the characteristic information.

The learning (training) may include, for example, supervised learning, unsupervised learning, and semi-supervised learning.

In the case of the supervised learning, training can be performed while comparing an image with the correct answer. If the correct answer is different from the objective target and the avoidance target found by the convolutional neural network, the learning can be performed in the direction of reducing the loss of a loss function. The loss function may include a binary cross entropy loss (BCEloss), etc., and an optimizer may use adam, RMSprop, and stochastic gradient descent. Also, the evaluation may be performed through a dice coefficient loss, etc.

When the objective target and the avoidance target are determined through the machine learning such as the convolutional neural network learning, the process proceeds to an operation (1230) to determine the final objective target.

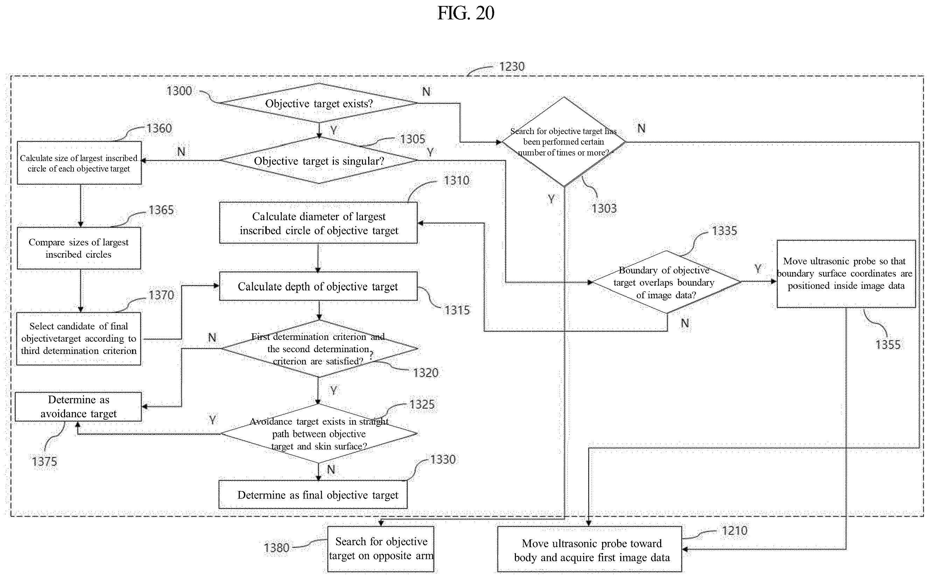

FIG. 20 illustrates a flowchart of specific processes of the operation (1230).

In an operation (1300), it is determined whether the objective target found in the operation (1225) exists. If the objective target exists, the process proceeds to an operation (1305) to determine whether the number of searched objective targets is one or two or more. First, the case where there is only one objective target will be described.

When there is only one objective target, it is determined whether the boundary of the objective target overlaps the boundary of the image data (operation (1335)). When the boundaries do not overlap each other, the process proceeds to an operation (1310), and when the boundaries overlap each other, the process proceeds to an operation (1355). The ultrasonic probe 41 is moved so that the boundary surface coordinates are positioned inside, for example, at the center of the image data, and the process returns to the operation (1210) to perform subsequent procedures. The body of a subject may be moved without moving the ultrasonic probe 41.

In the operation (1310), the diameter of the largest inscribed circle, which is one of the size-based information of the objective target, is calculated. The largest inscribed circle refers to an inscribed circle having the largest size among circles into which only pixels of the objective target can enter. In addition to the diameter of the largest inscribed circle, the size-based information of the objective target includes other information about the maximum and minimum lengths of horizontal and vertical lines that can be drawn within the objective target, the maximum and minimum distances from the center of gravity of the objective target to the boundary line of the objective target, the area of the objective target, and the like.

Subsequently, a depth, which is depth-based information about the objective target, is calculated (operation (1315)), and it is determined whether the objective target satisfies a first determination criterion and a second determination criterion (operation (1320)). The "depth-based information" includes not only simple depth, but also various other information related to the depth.

When the objective target is a superficial vein, the first determination criterion is a criterion for determining whether the diameter of the largest inscribed circle is greater than or equal to a certain value (first value). The first value may be determined within a device operation error range. For example, when the diameter of the largest inscribed circle is less than 2 mm, the objective target may be classified as an avoidance target (operation (1375)). When the diameter of the largest inscribed circle is greater than or equal to the first value, it is determined that the first determination criterion is satisfied.

When the size-based information is information on the lengths of horizontal lines and vertical lines that can be drawn in the objective target, the first determination criterion may be a criterion for determining whether each of the maximum and minimum lengths of the horizontal lines and the vertical line is greater than or equal to a certain value. For example, when the minimum length of the horizontal lines and the vertical lines is less than 2 mm, the objective target may be classified as the avoidance target. When the objective target is not singular, an objective target having a larger sum, product, average, etc. of the horizontal and vertical lines may be selected as a candidate for the final objective target (operation (1370)).

In an embodiment in which the size-based information is the maximum and minimum distances from the center of gravity of the objective target to the boundary line, a criterion for determining whether the maximum and minimum distances are greater than or equal to a certain value may be the first determination criterion. For example, when the minimum distance from the center of gravity of the objective target to the boundary line of the objective target is less than 2 mm, the objective target may be classified as the avoidance target. When there are a plurality of objective targets, an objective target having a larger sum, product, average, etc. of the distances from the center of gravity to the target boundary line may be selected as a candidate for the final objective target (operation (1370)).

In an embodiment in which the size-based information is information on area, a criterion for determining whether the area is greater than or equal to a certain value may be the first determination criterion. For example, when the area of the objective target is less than 10 mm², the objective target may be classified as an avoidance target. When there are a plurality of objective targets, an objective target having a larger area may be selected as a candidate for the final objective target (operation (1370)).

The second determination criterion is a criterion for determining whether the depth of the objective target is smaller than or equal to a certain value (second value), for example, 1 cm. When the depth of the objective target is greater than the second value, the objective target is determined as an avoidance target (operation (1375)). This is to exclude a deep vein that is not a superficial vein because hemostasis of the deep vein is more difficult than that of the superficial vein, and also to exclude an artery that is the avoidance target. In addition, when the depth is greater than the second value, the syringe needle may make a deep wound while entering, and there may be important anatomical structures or nerves within an invasive path. Therefore, the second determination criterion is applied. When the depth of the objective target is less than or equal to the second value, it is determined that the second determination criterion has been passed.

When the first determination criterion and the second determination criterion are satisfied, it is determined whether an avoidance target exists in the straight path between the objective target and the skin surface (operation (1325); a third determination criterion). When there is an avoidance target in the straight path, the avoidance target may be a nerve on the side of the arm.

When the determination result of the operation (1325) is "No", the corresponding objective target is determined as the final objective target (operation (1330)).

An objective target may be determined as the final objective target when satisfying all of the first to third determination criteria, but an objective target may be determined as the final objective target when satisfying at least one of the first to third determination criteria.

A case in which a plurality of objective targets have been searched for in the operation (1225) will be described.

When the plurality of objective targets have been searched for, the process proceeds to an operation (1360) to calculate the size of the largest inscribed circle for each of the objective targets. Then, the sizes of the largest inscribed circles are compared to each other (operation (1365)), and a candidate for the final objective target is selected according to the third determination criterion (operation (1370)).

The third criterion is as follows. In principle, the objective target having the largest size of the largest inscribed circle is selected as a candidate for the final objective target. However, when the difference in sizes between the largest inscribed circle and the inscribed circle of the objective target having the next largest inscribed circle is within a certain value (third value), for example, 100, an objective target close to the contact surface of the ultrasonic probe, for example, an objective target close to the center point of the contact surface of the ultrasonic probe, may be determined as a candidate for the final objective target.

Even when the size-based information other than the size of the largest inscribed circle is used as the first information, if the difference between the comparison values is within a certain value, an objective target close to the contact surface of the ultrasonic probe, for example, an objective target close to the center point of the contact surface of the ultrasonic probe, may be determined as a candidate for the final objective target.

When the candidate for the final objective target is determined in the operation (1370), the process proceeds to the operation (1315), and the subsequent processes are performed. Accordingly, the final objective target is determined, or the objective target may be determined as the avoidance target.

When it is determined in the operation (1300) that the objective target does not exist, it is determined whether the search for the objective target is performed a certain number of times or more. When the search has been performed a certain number of times, for example, 2 or 3 times or more, the process proceeds to an operation (1380) to search for an objective target with respect to the opposite arm. When the number of times is less than a certain number, the process proceeds to the operation (1210). The ultrasonic probe 41 is moved to acquire the first image data. In this case, the first image data is acquired by moving the ultrasonic probe 41 to a position that does not overlap with the previous image.

When the final objective target is determined by the above processes, the center coordinates of the final objective target are calculated in an operation (1235). The syringe needle unit 200 performs the invasive procedure at the center coordinates.

The center coordinates are determined as the coordinates of a point (x) having the maximum distance from any point (xᵢ) inside the largest inscribed circle that includes only the pixel of the final objective target.

After the calculation of the center coordinates is completed, when passing through the surface of the blood vessel a certain number of times, for example, two or more times within a straight path between the corresponding center coordinates and the skin surface, the corresponding final objective target may be excluded.

Also, circles having a certain diameter, for example 1 mm, are drawn for all pixels in the final objective target. Then, when a certain ratio of a pixel within the circle, for example 30 to 95%, preferably 50 to 70% or more, is classified as the final objective target, the corresponding pixel is determined as the final objective target, otherwise the corresponding pixel is deleted.

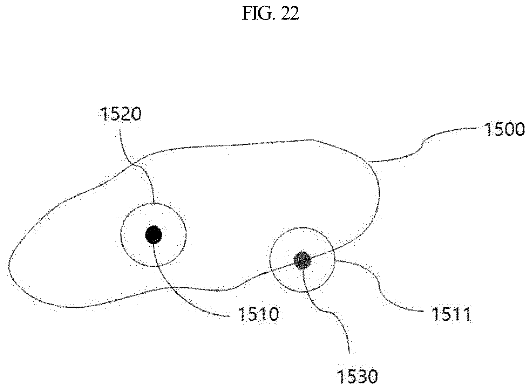

As illustrated in FIG. 22, the entirety (more than a certain ratio) of a pixel exists in a circle 1520 drawn on the basis of a pixel 1510, and thus, the corresponding pixel 1510 may be classified as a pixel of the final objective target. In a circle 1511 drawn on the basis of a pixel 1530, a pixel other than a pixel of the final objective target does not exist at more than a certain ratio, and thus, the corresponding pixel 1530 may be removed from the final objective target.

This process is intended to smooth the boundary of the final objective target and exclude the possibility of the presence of pixels to be misclassified. However, this process is not a process necessary for practicing the present invention, but a subsidiary process.

The present invention has been described above with reference to the accompanying drawings, but the scope of the present invention is determined by the following claims and should not be construed as being limited to the above embodiments and/or drawings. Also, it should be clearly understood that improvements, changes, and modifications obvious to those skilled in the art with respect to the invention described in the claims are also included in the scope of the present invention.

## Claims

1. An automatic invasive device for body comprising:
a syringe needle unit support part configured to support a syringe needle that enters the body; and
a press unit configured to press the body that has a site to be subjected to an invasive procedure.

2. The automatic invasive device for body of claim 1, wherein the press unit comprises:
a press band that comes into contact with and presses the body to be pressed;
at least one pair of moving units that support the press band; and
a driving device that drives the moving units in a pressing direction and in a press releasing direction.

3. The automatic invasive device for body of claim 2, wherein in an initial state prior to pressing, the press band is provided not to come into contact with the body to be pressed.

4. The automatic invasive device for body of claim 2 or 3, wherein the press unit comprises:
a guide bar;
a first nut that moves along the guide bar in a first direction by the operation of the driving device; and
a second nut that moves along the guide bar in a second direction opposite to the first direction,
wherein the first nut and the second nut are connected to the at least one pair of moving units and move the moving units along the guide bar.

5. The automatic invasive device for body of claim 4, further comprising a coupling that connects the guide bar to an output shaft of the driving device.

6. The automatic invasive device for body of any one of claims 1 to 3, further comprising:
an ultrasonic probe that senses a puncturing position; and
a probe unit that supports the ultrasonic probe and moves the ultrasonic probe in a certain direction.

7. A method for measuring blood pressure or pulse by using the automatic invasive device for body of claim 6, the method comprising:
a first operation of pressing the upper arm with the press unit; and
a second operation of measuring the blood pressure or pulse as the probe unit moves the ultrasonic probe in a certain direction in the lower arm and measures a blood flow rate by using an ultrasonic image.

8. The method of claim 7, further comprising a first-first operation of changing pressure applied on the upper arm.

9. The automatic invasive device for body of claim 1, further comprising a probe unit that comprises a probe for sensing a human body part to be subjected to an invasive procedure, a probe support part, and a driving device,
wherein the probe unit is rotated, by the operation of the driving device, in the width direction of the human body part to be subjected to the invasive procedure.

10. The automatic invasive device for body of claim 9, further comprising a direction-changing part for changing the driving force of the driving device into a rotatory force that enables rotation of the probe unit in the width direction.

11. The automatic invasive device for body of claim 9 or 10, wherein the probe unit further comprises a pressing part for pressing the target body.

12. The automatic invasive device for body of claim 11, wherein the probe unit further comprises an elastic member that connects the pressing part to the probe support part.

13. The automatic invasive device for body of claim 9 or 10, further comprising a linear driving device that moves the probe unit in the up-down direction.

14. The automatic invasive device for body of claim 1, further comprising an insertion member-driving part that drives an insertion member to be inserted into a syringe needle unit,
wherein the insertion member-driving part comprises:
a tube support part that supports the insertion member; and
a driving device that drives the insertion member support part.

15. The automatic invasive device for body of claim 14, wherein the insertion member support part comprises a gear tooth-shaped holder that comes into contact with and holds the insertion member.

16. The automatic invasive device for body of claim 15, wherein the gear tooth-shaped holder comprises a tooth portion that extends in the longitudinal direction of the insertion member support part.

17. The automatic invasive device for body of any one of claims 14 to 16, wherein the insertion member-driving part comprises:
a lead screw that receives the driving force of the driving device;
a nut that is rotated by the rotation of the lead screw and moves along the lead screw; and
a connection part which is connected to the nut and moved due to the movement of the nut, and is connected to the insertion member support part.

18. The automatic invasive device for body of claim 17, wherein the insertion member-driving part further comprises a guide that guides the connection part.

19. The automatic invasive device for body of claim 15 or 16, wherein the gear tooth-shaped holder rotates about a longitudinal axis of the insertion member support part.

20. The automatic invasive device for body of claim 1, further comprising a body contact material supply unit,
wherein the body contact material supply unit comprises:
a roller unit that supplies a body contact material having a roll shape; and
a press part that presses the body contact material so that the body contact material is brought into contact with the body at a certain position.

21. The automatic invasive device for body of claim 20, wherein the roller unit comprises:
a first roller on which a body contact material roll before use is wound;
a second roller on which a body contact material roll after use is wound; and
a driving device that rotates at least one of the first roller or the second roller.

22. The automatic invasive device for body of claim 21, wherein the roller unit further comprises at least one switching roller which is provided between the first roller and the second roller and changes a direction so that the body contact material moves in a certain direction.

23. The automatic invasive device for body of claim 22, wherein the switching roller comprises:
a first switching roller which is located on a downstream side of the first roller and changes a direction of a body contact material band supplied from the first roller;
a second switching roller which is located on a downstream side of the first switching roller and changes the direction of the body contact material band supplied from the first switching roller;
a third switching roller which is located on a downstream side of the second switching roller and changes the direction of the body contact material band supplied from the second switching roller;
a fourth switching roller which is located on a downstream side of the third switching roller and changes the direction of the body contact material band supplied from the third switching roller; and
a switching roller-driving device for driving at least one of the first to fourth switching rollers.

24. The automatic invasive device for body of claim 23, wherein the second switching roller and the third switching roller are arranged such that the body contact material band therebetween is placed above the body to be contacted.
